(19) **Europäisches Patentamt**
**European Patent Office**
**Office européen des brevets**

(11) **EP 2 002 808 A2**

(12) **EUROPEAN PATENT APPLICATION**
published in accordance with Art. 153(4) EPC

(43) Date of publication:
**17.12.2008 Bulletin 2008/51**

(51) Int Cl.:
***A61F 2/82*** *(2006.01)*

(21) Application number: **07738322.2**

(86) International application number:
**PCT/JP2007/054850**

(22) Date of filing: **12.03.2007**

(87) International publication number:
**WO 2007/122901 (01.11.2007 Gazette 2007/44)**

(84) Designated Contracting States:
**DE FR**

(30) Priority: **31.03.2006 JP 2006099548**

(71) Applicant: **Zeon Corporation
Tokyo 100-8246 (JP)**

(72) Inventors:
• **SHINOHARA, Naoki
Chiyoda-ku, Tokyo 100-8246 (JP)**

• **GON, Chimyon
Chiyoda-ku, Tokyo 100-8246 (JP)**
• **ASAHINA, Takumi
Chiyoda-ku, Tokyo 100-8246 (JP)**
• **TOYOKAWA, Yoshihide
Chiyoda-ku, Tokyo 100-8246 (JP)**

(74) Representative: **Beckmann, Claus
Kraus & Weisert
Patent- und Rechtsanwälte
Thomas-Wimmer-Ring 15
80539 München (DE)**

(54) **STENT DELIVERY CATHETER**

(57)  The present invention provides a stent delivery catheter comprising: an inner tube which has a distal end and a proximal end and in which a stent is set around the outer circumferential surface at the distal end side of the inner tube; an outer tube having a distal end and a proximal end, being coaxially arranged around the outer circumference of the inner tube, and being relatively movable in the axial direction to the inner tube; and a drive means being arranged at the proximal end side of the inner tube and the outer tube and being capable of making the outer tube relatively movable towards the proximal end to the inner tube, wherein with the operation of the drive means, a liquid fluidly travels through the gap between the inner tube and the outer tube from the proximal end side to the distal end direction.

Fig. 2

**EP 2 002 808 A2**

## EP 2 002 808 A2

**Description**

Technical Field

The present invention relates to a stent delivery catheter to be used for indwelling a stent in a body. More specifically, the present invention relates to a stent delivery catheter which can be easily operated for indwelling a stent in a body.

Background Art

In order to secure persistence of digestive organ narrowed by cancer cells or of blood vessel narrowed by arteriosclerosis, for example, a method of medical treatment by indwelling a stent into the narrowed section is known. The stent is a device to be indwelled so as to secure the lumen by expanding itself at the narrowed section. Depending on the expanding method, it is roughly categorized into a self-expandable stent and a balloon-expandable stent. These may be chosen depending on the region where narrowing is caused; when indwelling particularly a self-expandable stent in the body, for instance, use of a stent delivery catheter described in Patent Documents 1 to 8 is known. It should be noted that Patent Document 2 is the publication of foreign application corresponding to the Patent Document 1, Patent Document 4 is the publication of foreign application corresponding to the Patent Document 3, Patent Document 6 is the publication of foreign application corresponding to the Patent Document 5, and Patent Document 8 is the publication of foreign application corresponding to the Patent Document 7.

The stent delivery catheters described in Patent Documents 1 to 8 are respectively configured with coaxially-arranged two tubes (an inner tube and an outer tube). A catheter is inserted into a body in a condition where a self-expandable stent is set between the inner tube and the outer tube; then, the stent is released at a position to be indwelled by pulling the outer tube to the direction of outside of the body (proximal end side) so as the stent to be indwelled in the body.

In case of the above-described conventional stent delivery catheter, friction and catch have been caused between outer circumferential surface of the stent set around the inner tube and inner surface of the outer tube, or between outer circumferential surface of the inner tube and inner circumferential surface of the outer tube. Friction and catch have been obstacles when pulling the outer tube to the proximal end side; thereby operators have often had trouble with subtle adjustment of the position. As a countermeasure, as described in e.g. Patent Documents 1 and 2, a method for reducing friction by applying a lubricant to a portion of a stent delivery catheter where friction may be caused. However, considering circumstances where the lubricant may remain in the body of the patient and the lubricant may adhere to other surgical instruments and the like which may hinder the use thereof, usage of lubricant is restricted; thus sufficient effect cannot always obtained.

Moreover, in the clinical practice, in order to prevent friction and catch, repeating an operation for connecting a syringe filled with normal saline to an injection port provided in the side wall of a stent delivery catheter and pulling an outer tube into the proximal end direction, and an operation for supplying normal saline from the syringe into the gap between the inner tube and the outer tube has been carried out.
Nevertheless, the supplied normal saline between the inner tube and the outer tube with pulling operation of the outer tube is immediately lost, normal saline has to be frequently supplied. Whereby, operation during the surgery has been extremely complex. As a result, duration of surgery becomes prolonged, which increases physical strain given to the operators and the patients.

  Patent Document 1: Japanese Patent Application Laid-Open (JP-A) No.2004-223262
  Patent Document 2: US Patent Application Laid-Open No. 2004/0148007, specification
  Patent Document 3: JP-A No. 10-57502
  Patent Document 4: European Patent Application Laid-Open (EP-A) No.0819411, specification
  Patent Document 5: JP-A No. 11-313893
  Patent Document 6: US Patent 6,425,898
  Patent Document 7: JP-A No. 2001-37885
  Patent Document 8: EP-A No. 1064888, specification

Disclosure of the Invention

Problems to be solved by the Invention

Accordingly, an object of the present invention is to provide a stent delivery catheter which inhibits friction and catch between the inner tube and the outer tube but also enables to easily indwell a stent at an operator's intended position in the patient's body by smoothly moving the outer tube to the proximal end side for indwelling the stent in the body, so as to solve the above problems.

Means for Solving the Problems

In order to solve the above problems, the present invention provides a stent delivery catheter comprising: an inner tube which has a distal end and a proximal end and in which a stent is set around the outer circumferential surface at the distal end side of the inner tube; an outer tube having a distal end and a proximal end, being coaxially arranged around the outer circumference of the inner tube, and being relatively movable in the axial direction to the inner tube; and a drive means being arranged at the proximal end side of the inner tube and the outer tube and being capable of making the outer tube relatively movable towards the proximal end to the inner tube, wherein with the operation of the drive means, a liquid fluidly travels through the gap between the inner tube and the outer tube from the proximal end side to the distal end direction.

In this aspect of the invention, the stent delivery catheter preferably comprises a housing which incorporates the proximal end of the inner tube and the proximal end of the outer tube, in which the outer tube is relatively movably inserted in the axial direction, and to which the drive means is mounted, wherein, in the housing, a cylinder for reserving the liquid is arranged, and a plunger is relatively movably arranged to the cylinder in the axial direction of the cylinder and fixed to the outer tube, when the outer tube moves relatively to the inner tube in the proximal end by the motion of the drive means, the plunger presses the liquid in the cylinder; thereby the liquid travels, by the pressure, through the gap between the inner tube and the outer tube from the proximal end side to the distal end direction.

Alternatively, the stent delivery catheter preferably comprises a housing which incorporates the proximal end of the inner tube and the proximal end of the outer tube, in which the outer tube is relatively movably inserted in the axial direction, and to which the drive means is mounted, wherein the drive means comprises: a drive shaft fixed at a proximal end portion of the outer tube and being relatively movable in the axial direction to the housing; a drive plate having a through-hole, being movably held in the axial direction to the drive shaft by inserting the drive shaft into the through-hole, and being latched to the drive shaft by tilting towards the axial core of the drive shaft; and a drive lever which is pivotaly mounted around a fulcrum located at a position certain distance away from the axial core of the housing and which is pressed, by pivoting, towards the proximal end side of the drive plate, wherein the drive lever makes the drive plate be tiltedly latched onto the drive shaft by pressing the drive plate towards the proximal end side, and further makes both the drive shaft and the outer tube fixed to the drive shaft be moved towards the proximal end side together with the drive plate.

Further, in the above aspect of the invention, preferably, the inner tube comprises a cylindrical inner tube body and a projection arranged at the distal end side of the inner tube body and projecting from the distal end of the outer tube, the inner tube body comprises a cylindrical body portion and a thin diameter part arranged at the distal end side of the body portion and formed in a manner thinner than the diameter of the body portion for arranging the stent, the projection comprises: a taper portion which is continuously arranged with the thin diameter part and has a taper expanding the radius relative to the distal end direction; and an apical portion located at the distal end side of the taper portion, on the surface of the taper portion, groove extending along the axial direction of the inner tube is formed.

In the above preferable aspect of the invention where groove is formed therein, preferably, the groove is formed such that the width is widened towards the proximal end direction and is continuously extended from the distal end to the proximal end of the taper portion.

In the above preferable aspect of the invention where groove is formed therein, preferably, the groove is plurally provided, a plurality of the grooves are arranged at regular intervals in the circumference direction relative to the axial core of the inner tube body as the center, further a plurality of the grooves are in the same shape.

In the above preferable aspect of the invention where groove is formed therein, the inner tube body and the projection are independent from each other.

Still further, preferably, the outer tube comprises: a resin layer made of a polymer material; and a tubular braided body which is formed by helically-braided plurality of wire rods and buried in the resin layer, wherein, when the braided body is planarily seen, an angle $\alpha$ (°) between the wire rod and the axis line of inner tube can be determined by the following relational expression.

$$35 < \alpha < 60$$

in this case, the braided body is preferably arranged in the position other than the position corresponding to the stent setting position in the inner tube.

Effects of the Invention

The stent delivery catheter of the present invention, with the operation for indwelling a stent in the body, is capable of supplying liquid to the contact portion of the inner circumferential surface of the outer tube and the outer circumferential surface of the inner tube. As a result, by the lubricating effect of the supplied liquid, friction and catch are hardly caused between the inner tube and the outer tube; thereby operation for indwelling a stent in the body can become easier. Moreover, when the stent delivery catheter of the present invention is used for a self-expandable stent, the liquid is supplied from outside of the body every time the pulling operation of the outer tube is carried out, so the stent is cooled and its expansive force is also weakened; thus, it is possible to reduce friction between the stent and the outer tube.

In addition, about another aspect of the stent delivery catheter wherein at least one groove is formed on the taper surface of the projection of the inner tube, since taper portion is arranged to continue into the thin diameter part, catching by the inner tube to the stent at a time of withdrawal of the catheter from the body can be prevented. Still further, as at least one groove is provided to the taper portion, contact area of taper portion with the edge of distal end opening of the outer tube covering the surface of the taper portion decreases, adhesiveness between the inner tube and the outer tube but also withdrawal resistance of the outer tube is reduced; hence, it is capable of smoothly indwelling the stent. Namely, according to the aspect of the invention, a phenomenon where the inner tube and the outer tube are closely contact each other and another phenomenon where stent is caught to the inner tube when the inner tube is withdrawn from the released stent are prevented simultaneously. Consequently, a stent delivery catheter which is capable of easily indwelling a stent to an intended position can be provided.

In still further aspect of the stent delivery catheter where the outer tube comprises a resin layer and a braided body, even if the outer tube is made thinner, it can keep sufficient stiffness; thereby checking the position of indwelled stent by endoscope can be easily done.

Brief Description of the Drawings

Fig. 1(a) is a side view showing an embodiment of the stent delivery catheter 100 of the present invention, and Fig. 1(b) is a cross-sectional view also showing the embodiment of the stent delivery catheter 100 of the invention taken along the line X-X' of FIG. 1(a);
Fig. 2 is a cross-sectional view showing an operating portion 100b of the stent delivery catheter 100;
Fig. 3 is an enlarged sectional view of the operating portion 100b;
Fig. 4(a) is an enlarged side view showing a projection 13 of the stent delivery catheter 100, and Fig. 4(b) is an enlarged plan view of the projection 13 seen from a direction indicated by arrow A;
Fig. 5 (a) is a perspective view showing projection 130 of another embodiment of the invention, Fig. 5(b) is a plan view of the projection 130 seen from a direction indicated by arrow B, Fig. 5(c) is a side view of the same, and Fig. 5(d) is a plan view of the same seen from a direction indicated by arrow C;
Fig. 6 is a perspective view showing an outer tube 20; and
Fig. 7 is an enlarged plan view of a braided body 20b.

Description of the reference numerals

| | |
|---|---|
| 10 | inner tube |
| 11 | inner tube body |
| 11a | thin diameter part |
| 11b | body portion |
| 13, 130 | projection |
| 13a, 130a | apical portion |
| 13b, 130b | taper portion |
| 13c, 130c | groove |
| 20 | outer tube |
| 20b | braided body |
| 24 | resin layer |
| 40 | stent |
| 50 | housing |

| 58 | cylinder |
| 60 | drive lever |
| 64 | rotational axis (fulcrum) |
| 70 | drive shaft |
| 72 | drive plate |
| 72a | through-hole |
| 80 | plunger |
| 100 | stent delivery catheter |
| 100a | catheter portion |
| 100b | operating portion |

Best Mode for Carrying Out the Invention

In order to make the understanding of the present invention easier, the present invention will be described in detail with reference to the numerals indicated in the attached drawings. However, the present invention is not limited to by the embodiment shown in the drawings.

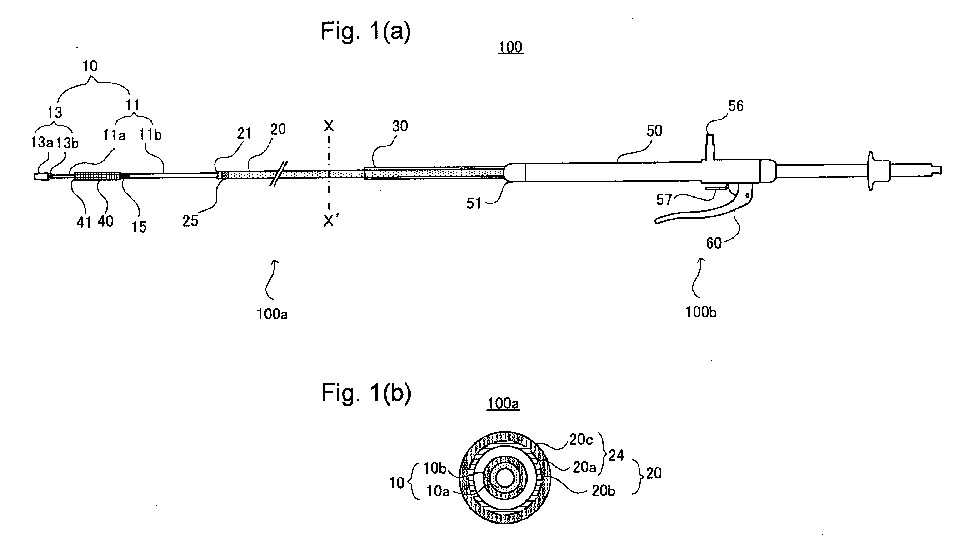

Fig. 1(a) is an entire side view showing the stent delivery catheter 100 (hereinafter, it may be simply referred as "catheter 100".) as an embodiment of the present invention. Fig. 1(b) is a cross-sectional view of the embodiment taken along the line X-X' of FIG. 1(a). The catheter 100 comprises a catheter portion 100a to be inserted into the body, and an operating portion 100b which is connected to the catheter portion 100a and used for operation of the catheter portion 100a in the body from outside the body.

As shown in Figs. 1(a) and 1 (b), the catheter portion 100a comprises: an inner tube 10 having a distal end and a proximal end; and an outer tube 20 having a distal end and a proximal end.

The outer tube 20 is arranged around the outer circumferential side of the inner tube 10 so as to be concentric with the inner tube 10. Between the outer circumferential surface of the inner tube 10 and the inner circumferential surface of the outer tube 20, a certain gap is given. The outer tube 20 is, by the operation of below-described operating portion 100b, relatively movable in the axial direction relative to the inner tube 10. When the catheter portion 100a is inserted into the patient's body, a stent 40 is set such that it covers the outer circumferential surface of distal end side of the inner tube 10, and the outer tube 20 is arranged such that the outer tube 20 covers the outer circumferential surface of the stent 40.

For easy understanding of the internal structure of catheter portion 100a, Fig. 1(a) illustrates a state where the stent 40 is exposed by the movement of the outer tube 20 to the proximal end side by a catheter operation. In the catheter 100 before actual stent indwelling operation, distal end 21 of the outer tube 20 is located closer to the distal end side than distal end 41 of the stent 40. The outer tube 20 controls the expansion of the stent 40 in the radial direction by being arranged to cover the entire surface of the stent 40 and a taper portion 13b which locates at the opening of the distal end of outer tube 20 but also locates in the projection 13 of the inner tube 10.

In the embodiment shown in the figure, it should be noted that a non-expanded stent 40 is shown in order to make clear the positional relationship among the stent 40, the inner tube 10, and the outer tube 20. With the proviso that a case where the stent 40 is self-expandable stent, once the stent 40 is released from the outer tube 20 (see Fig. 1(a)), the stent 40 expands by itself and detaches from the outer circumferential surface of the inner tube 10.

The catheter portion 100a of the embodiment of the invention has an outermost tube 30 arranged concentrically with the outer tube 20 so as to cover the outer circumferential surface of the outer tube 20 at the proximal end side of the outer tube 20. Moreover, at the vicinity of the distal end of the inner tube 10 and outer tube 20, radiopaque markers 15, 25 are respectively mounted.

The inner tube 10 comprises a cylindrical inner tube body 11 and a projection 13 arranged at the distal end of inner tube body 11 and projecting from the distal end of outer tube 20 at a time of stent indwelling operation. The inner tube body 11 comprises a cylindrical body portion 11b and a thin diameter part 11a arranged at the distal end side of the body portion 11b and formed into a thinner diameter, for setting the stent 40, than that of the body portion 11b.

The projection 13 is arranged to continue to the thin diameter part 11a. The projection 13 comprises a taper portion 13b having a taper expanding the radius relative to the distal end direction and a apical portion 13a located at the distal end side from the taper portion 13b. The taper portion 13b of the projection 13, in a state before stent indwelling operation, is covered by an edge of distal end opening of the outer tube 20.

The inner tube 10, as shown in Fig. 1(b), comprises a lumen for inserting a guide wire. By inserting a guide wire to the lumen, the inner tube 10 is capable of inserting the catheter portion 100a to a targeted part of the body along the guide wire.

In the embodiment, the inner tube body 11, at the proximal end side from the stent 40 mounting position, is in a double-layered structure consisting of an inner-tube inner layer 10a and an inner-tube outer layer 10b formed to cover the inner-tube inner layer 10a. Distal end of the inner-tube inner layer 10a extends to the distal end side from the distal end of the inner-tube outer layer 10b; thereby a thin diameter part 11a for mounting the stent 40 is formed. A step formed at the border between the thin diameter part 11a and the distal end of the inner-tube outer layer 10b works as a stopper for preventing the stent 40 from moving towards the proximal end side together with the outer tube 20 when the outer tube 20 is moved to the proximal end side as a necessary operation for indwelling the stent 40 to a predetermined position.

Material of the inner tube 10 is not specifically limited to; it is desirably flexible and is required to have certain stiffness as well as slidability. Therefore, a synthetic resin is normally used. In the embodiment, material of the inner-tube inner layer 10a is a high-stiffness polyether ether ketone resin; meanwhile material of the inner-tube outer layer 10b is a high-density polyethylene which exhibits excellent slidability. For the improvement of stiffness and slidability, coating or the like may be given on the surface of the material. Dimension of the inner tube 10 is normally about a length which totaled the length of the outer tube 20 and that of the operating portion 100b; the outer diameter of the tube is about 0.5 to 3.0 mm.

The outer tube 20, as seen from Fig. 1(b), has a slightly larger inner diameter than outer diameter of the inner tube 10. Between outer circumferential surface of the inner tube 10 and inner circumferential surface of the outer tube 20, a gap is formed for setting the stent 40. Material of the outer tube 20 is not particularly restricted to, in view of demand for flexibility; synthetic resin and elastomer are usually used. These materials may be reinforced by metal wire and so on, or the surface may be coated. The outer tube 20 in the catheter 100 of the shown embodiment comprises: a resin layer 24 having a double-layered structure consisting of the inner layer 20a and the outer layer 20c; and the braided body 20b buried in the resin layer 24. Dimension of the outer tube 20 is normally about 300 to 2500 mm in length, 0.8 to 3.0 mm in inner diameter, and 1.0 to 4.0 mm in outer diameter. Structure of the outer tube 20 will be described in detail as below.

An outermost tube 30 is a tube arranged concentrically with the outer tube 20 so as to cover outer circumferential surface of the certain length of outer tube 20 from the operating portion 100b in the distal end direction. The proximal end side of the outermost tube 30 is fixed with a tip of the front cap 51 of the housing 50 in the below-described operating portion 100b. In the embodiment, the outermost tube 30 is not an essential element; however, if the outer tube 20 becomes the outermost layer of the catheter portion 100a, it becomes impossible to hold the outer tube 20 by hand at a time of moving of the outer tube 20 to the proximal end side as an indwelling operation of the stent 40. Because, if the outer tube 20, which attempts to move towards the proximal end, is held by hand and fixed at the position, inner tube 10 eventually moves to the distal end direction relative to the inner-wall of the body. Thereby indwelling position of the stent 40 tends to be misaligned. Accordingly, in order to make the catheter 100 handheld and to inhibit misalignment of indwelling position of the stent 40, providing an outermost tube 30 as shown in the figure is preferable. The outermost tube 30 to be provided is usually 500 to 1500 mm shorter than the outer tube 20, and the inner diameter of the outermost tube 30 is about 0.05 to 1.0 mm larger than the outer diameter of the outer tube 20. The material is not restricted to but is normally synthetic resin, in the shown catheter 100, polyacetal is used.

Radiopaque markers 15, 25, which are mounted at the vicinity of the distal end of the inner tube 10 and the outer tube 20, are a label of which position in the body is detected by the fluoroscope. These are formed by polymer and the like in which metal material such as gold, platinum, and tungsten together with barium sulfate and bismuth oxide are blended. When radiopaque markers 15, 25 are mounted at the vicinity of the distal end of inner tube 10 and/or outer tube 20, position of the radiopaque markers 15, 25 can be fluoroscopically detected; thereby it is possible to precisely track the position of the apical portion of the catheter 100, hence it is preferable.

Particularly, as shown in the figure, when radiopaque markers 15, 25 are respectively mounted to the inner tube 10 and the outer tube 20, it is possible to know whether the stent 40 is released or not by tracking relative position of individual tubes, thus it is preferable. Shape of the radiopaque markers 15, 25 is not limited to; it is preferably ring shape. In addition, radiopaque markers 15, 25 may be mounted on the inner circumferential surface or outer circumferential surface of the inner tube 10 and/or the outer tube 20; these also may be buried in the inner tube 10 and/or the outer tube 20. In the Embodiment, as mentioned above, distal end of the inner-tube outer layer 10b works as a stopper of the stent 40; however, when the inner tube 10 is constituted by a single layer, by forming a step by mounting the radiopaque marker 15 onto the outer circumferential surface of the inner tube 10, it is possible to make the radiopaque marker 15 work as a stopper.

When the catheter 100 is inserted into the body, and then the below-described drive lever 60 of the operating portion 100b is operated, the outer tube 20 relatively moves a certain distance, relative to the inner tube 10, towards proximal end side in a manner to be pulled through inside the operating portion 100b. As a result, a part of distal end side of the stent 40, which has been completely covered by the outer tube 20 and mounted around the outer circumferential surface of the inner tube 10, is exposed. Then, by repeating the above operation of the drive lever 60, the outer tube 20 can be intermittently moved towards the proximal end side and the stent 40 can be completely exposed around the outer circumferential surface of the inner tube 10.

Operation of the drive lever 60 of the operating portion 100b, in accordance with the below-mentioned mechanism, also provides liquid from the operating portion 100b to the catheter portion 100a. With the movement of the outer tube 20 towards the proximal end side by the operation of drive lever 60, liquid is supplied from the operating portion 100b (in other words, proximal end side of the catheter portion 100a) to the gap between the inner tube 10 and the outer tube 20. Consequently, friction caused between the outer circumferential surface of the inner tube 10 and the inner circumferential surface of the outer tube 20 can be reduced. So, each time when the drive lever 60 is operated, movement of the outer tube 20 towards the proximal end side and supply of liquid happened simultaneously; by repeating these operations, eventually, the stent 40 will be completely exposed, from the outer tube 20, at an accurate position where the operator is intended and be indwelled in the body by expanding outward on its own.

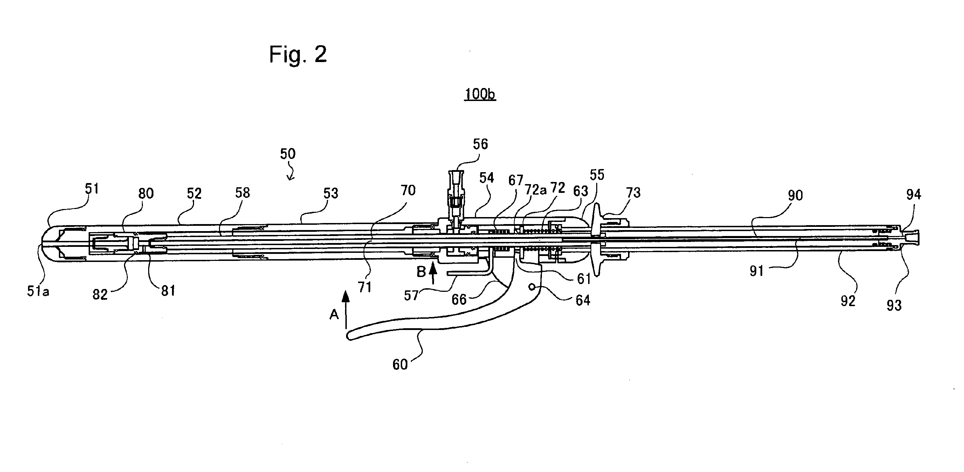

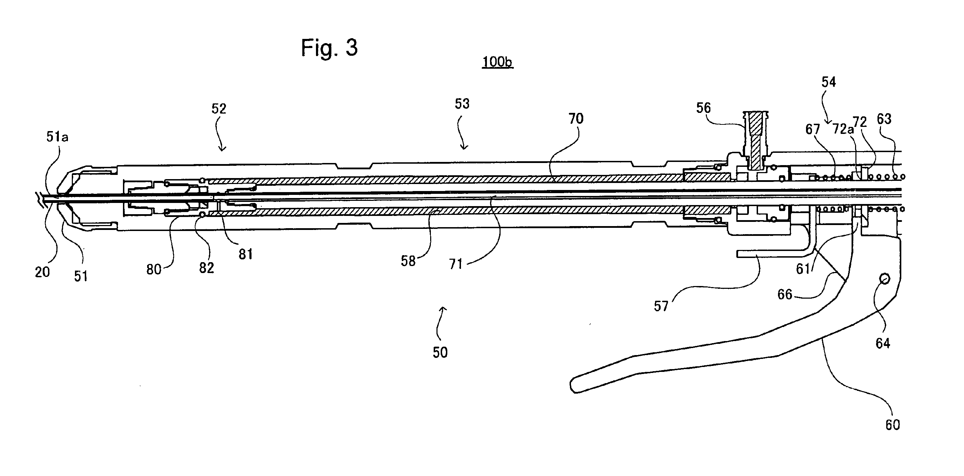

Fig. 2 is a cross-sectional view showing the operating portion 100b of the stent delivery catheter 100. Also, Fig. 3 is an enlarged sectional view of distal end side of the operating portion 100b of the stent delivery catheter 100. In Fig. 2, the inner tube 10 and the outer tube 20 are omitted; meanwhile, in Fig. 3, the inner tube 10 is omitted. Moreover, in Fig. 3, for the understanding of position of liquid in the below-described housing 50, the liquid in the housing 50 is shown by a hatched pattern. Hereinafter, motion of the operating portion 100b will be described in detail with reference to Figs. 2 and 3.

Each proximal end side of the inner tube 10 and the outer tube 20 of the above-described catheter portion 100a is concentrically introduced to inside of the operating portion 100b. The operating portion 100b comprises a housing 50 arranged concentrically with the inner tube 10 and so on.

The housing 50 is formed by connecting, in the order from distal end side, each block of: a front cap 51, a first intermediate cylinder 52, a second intermediate cylinder 53, an operation cylinder 54, and a rear cap 55. So, the housing 50 forms a capsule-shape of which schematic inside is hollow. To the distal end side of the front cap 51, the proximal end side of the above-mentioned outermost tube 30 is fixed; at the vicinity of the axis, a hole 51a which allows the inner tube 10 and the outer tube 20 to introduce into the housing 50 is provided.

The operation cylinder 54 is thickly formed than the thickness of blocks constituting the other housings. To the operation cylinder 54, the drive lever 60 is pivotally mounted. A fulcrum 64 of the pivoting is determined at a position where is certain distance apart from the operation cylinder 54 in the radial direction. To the operation cylinder 54, still, a liquid injection port 56 and a reverse switch 57 both described below are provided.

In the hollow inside the housing 50, proximal end portion of the outer tube 20 is inserted through the hole 51a of the front cap 51. To the proximal end portion of the outer tube 20, a cylindrical drive shaft 70 having a lumen 71 is fixed through a below-mentioned plunger 80, and a lumen of the outer tube 20 is communicated with the lumen 71 of the drive shaft 70. Moreover, a certain space is provided between the outer circumferential surface of the drive shaft 70 and the inner circumferential surface of the housing 50 to form a cylindrical space. This space is used as a cylinder 58 for reserving a liquid to be supplied between the inner tube 10 and the outer tube 20. Also, the cylinder 58 is communicated with the liquid injection port 56.

At the distal end of the drive shaft 70, a plunger 80 having a lumen is mounted, and a lumen 71 in the drive shaft 70 is communicated with the lumen of the plunger 80. The outer circumferential surface of the plunger 80 and the inner circumferential surface of the housing 50 are slidably abutted against each other. At the distal end of the plunger 80, the outer tube 20 is mounted to be inserted into the lumen of the plunger 80. Further, in the plunger 80, a hole 81 is formed as a through-hole for communicating the cylinder 58 with the lumen of the outer tube 20 mounted to the plunger 80 and making the liquid travel through the gap between the inner tube 10 and the outer tube 20.

When the stent delivery catheter 100 of the present invention is used, the cylinder 58 is filled with liquid such as normal saline through liquid injection port 56; later, the liquid injection port 56 is sealed. Then, by the operation of drive lever 60, the drive shaft 70 is moved towards the proximal end direction, the plunger 80 imparts pressure to the liquid which fills the cylinder 58 with. Hence, the liquid in the cylinder 58 flows into the gap between the inner tube 10 and the outer tube 20 through the hole 81 and the lumen 71 of the drive shaft 70 and travels through the catheter portion 100a in the distal end direction, finally the liquid is discharged from the distal end of the catheter portion 100a into outside (the body).

In addition, so as to prevent leakage of the liquid from the gap between the outer circumferential surface of the plunger 80 and the inner circumferential surface of the housing 50 in the distal end direction, an O-ring 82 is arranged at the outer side of the plunger 80. Moreover, in order to prevent the liquid which has flown from the hole 81 into the gap between the inner tube 10 and the outer tube 20 in the proximal end direction, the gap between the inner tube 10 and the outer tube 20 in the lumen 71 of the drive shaft 70 is sealed in the axial direction proximal end side of the hole 81.

With the above configuration, when the stent 40 is indwelled in the body, liquid is supplied between the inner tube 10 and the outer tube 20 both of which are relatively moved in the axial direction so that friction between them declines; thereby operation of stent indwelling can be easily carried out.

The operating portion 100b comprises the above drive shaft 70 and a drive plate 72 inside the operation cylinder 54, wherein the drive plate 72 is fitted to the drive shaft 70 by having the through-hole 72a and inserting the drive shaft 70 into the through-hole 72a.

The drive shaft 70 is movably inserted inside the housing 50 in the axial direction and the distal end of the drive shaft 70 is fixed to the proximal end side of the plunger 80. On the other hand, proximal end side of the drive shaft 70 is configured to be slidable and protrudably from an end hole of the rear cap 55 to the outer proximal end side; then, a below-described manual grip 73 is fixed at the proximal end of the shaft.

Inside the drive shaft 70, a slide guide pipe 90 is inserted. In addition, so as to make the relative movement to the slide guide pipe 90 possible, the lumen 71 is formed inside the drive shaft 70. In the same manner as the drive shaft 70, inside the slide guide pipe 90, a lumen 91 is formed for inserting a guide wire.

To the rear cap 55 located at the proximal end side of the housing 50, a cylindrical pipe cover 92 is mounted towards the proximal end direction. Moreover, at the proximal end side of the pipe cover 92, a pipe cap 93 is fitted; in the pipe cap 93, a through-hole 94 is provided for allowing a guide wire to penetrate therein in the axial direction. Into the distal end side of the through-hole 94, the proximal end side of the slide guide pipe 90 is inserted and fixed. In other words, proximal end side of the slide guide pipe 90 is fixed to the pipe cap 93; meanwhile, the distal end side of the same is slidably inserted in the lumen 71 of the drive shaft 70. The slide guide pipe 90 is arranged inside the pipe cover 92.

In the lumen 71 of the drive shaft 70, when the distal end of the slide guide pipe 90 is connected with the proximal end of the inner tube 10, a through-hole is continuously formed from the proximal end of the slide guide pipe 90 to the distal end of the inner tube 10, which allows a guide wire to be inserted into the through-hole for guiding the catheter 100 from outside the body to the stent indwelling position. Because of this composition, the slide guide pipe 90 and the inner tube 10 connected thereto are fixed to the housing 50.

To the pipe cover 92, a manual grip 73 is slidably mounted in the axial direction around the outer circumferential surface. The manual grip 73 is formed in a disk-shape to cover the circumference of the pipe cover 92 and is directly connected to the proximal end of the drive shaft 70 through slit arranged in the axial direction at the upper and lower part of the pipe cover 92. Therefore, by the manual grip 73, it is possible to relatively transport the drive shaft 70 around the slide guide pipe 90 in the axial direction.

The drive plate 72 arranged at the circumferential side of the drive shaft 70 is relatively movably fitted to the drive shaft 70 in the axial direction by inserting the drive shaft 70 into the through-hole 72a of which inner diameter is slightly larger than the outer diameter of the drive shaft 70 incorporated in the drive plate 72. Moreover, an end of the pressure spring 63 abuts to the drive plate 72; when no external force acts to the drive lever 60, the drive plate 72 is supposed to still stand at a position substantially perpendicular to the axial core of the drive shaft 70 by the act of the pressure spring 63. In such a condition, to the through-hole 72a of the drive plate 72 there is a little gap around the outer circumference of the drive shaft 70; so, if the below-described reverse switch 57 is ignored, the drive shaft 70 is movable in the axial direction to the drive plate 72.

The drive lever 60 is integrally formed with the drive projection 61 abutting to the circumferential portion of the drive plate 72 in the rear anchor side of the drive lever 60. It is pivotally arranged in a direction shown by arrow A in Fig. 2 using a pivotal axis 64 engaged between a pair of projection 66 formed in the outer circumference of the housing 50 as a fulcrum.

When the drive lever 60 is pivoted to the arrow A direction, the drive projection 61 presses the drive plate 72 towards the proximal end side and the drive plate 72 is tilted to the axis of the drive shaft 70 to latch together with the circumference of the drive shaft 70. Further, when pivoting the drive lever 60 in arrow A direction, the drive lever moves the drive plate 72 towards the proximal end side against the elastic force of the pressure spring 63, the drive shaft 70 to which the circumference the drive plate 72 latches moves towards the proximal end side simultaneously. As a result, the outer tube 20 connected to the drive shaft 70 also moves towards the proximal end side which is a direction where the outer tube 20 is withdrawn into the housing 50.

When releasing the holding force of the drive lever 60, the drive plate 72 is moved back to the initial position by the action of spring 63 abutting to the drive plate 72; at the same time, the drive lever 60 is also pivoted to move back to its initial position. At this time, since the drive plate 72 moves back to the substantially perpendicular position to the axial core of the drive shaft 70, when the drive plate 72 is pushed backwards, latch to the circumference of the drive shaft 70 is released and the drive plate 72 relatively movable to the drive shaft 70 so that the drive shaft 70 is never returned to the initial position.

When holding the drive lever 60 once again and pivoting it into the arrow A direction again, the drive shaft 70 further moves towards the proximal end side by the same operation as described above, and the outer tube 20 connected to drive shaft 70 also moves together with the drive shaft 70. By repeating the pivoting operation of this drive lever 60, the outer tube 20 gradually moves to the proximal end side, and the stent 40 is gradually exposed from the distal end side. Finally, when the outer tube 20 completely comes off from the stent 40, the stent 40 restrained by the outer tube 20 is released to expand itself.

In the embodiment, movement of the outer tube 20 in the proximal end direction, necessary for stent indwelling, is carried out only by repeating the above pivotal operation of the drive lever 60. As for the conventional stent delivery catheter making the outer tube move by directly pulling the tube in the axial direction towards the proximal end, when pulling the outer tube, the pulling force is also applied to the inner tube to be fixed in the axial direction so that there is a problem where indwelling position of the stent tends to be easily misaligned. Pivot of the drive lever 60, which is the stent indwelling operation of the embodiment, is a motion to apply a force in the perpendicular direction to the axial direction as a moving direction of the outer tube 20; therefore, when operating the drive lever 60, no force is added in the axial direction. Hence, changing directions for applying a force enable to solve the problem of misalignment regarding the stent indwelling position by the conventional moving operation of the outer tube. Moreover, the structure which makes the drive plate 72 tilt by the pivot of the drive lever 60 can be seen as a "leverage" having the drive lever 60 as a point of force, and the drive projection 61 for tilting the drive plate 72 as a point of action. Using the drive lever 60 applying the leverage for moving the outer tube 20 has an effect for lower the necessary force used for the operation when pulling force of the outer tube 20 must be higher such as a case of moving the outer tube 20 in a complicatedly-winding celom.

Causing the movement of the outer tube 20 to the proximal end side and supply of the liquid into the gap between the inner tube 10 and the outer tube 20 at the same time by the operation of drive lever 60 means that the liquid travels through the gap between the inner tube 10 and the outer tube 20 at a moment when friction force is applied between the inner tube 10 and the outer tube 20 by the movement of the outer tube 20; namely, it means that the liquid travels between the inner tube 10 and the outer tube 20 at a moment when lubricity is most required. So, if the stent delivery catheter 100 of the present invention is used, friction between the inner tube 10 and the outer tube 20 can be effectively lowered, or movement of the outer tube 20 to the proximal end side can be smoothly carried out without causing any catch between the outer tube 20 and the inner tube 10. In the same manner as the above, the liquid is also supplied to the gap between the outer tube 20 and the stent 40; even in relation to the stent 40, the outer tube 20 can be smoothly moved to the proximal end side.

The material which constitutes the operating portion 100b of the stent delivery catheter 100 of this embodiment may be arbitrary metal or synthetic resin and may not be particularly restricted to. With respect to the drive shaft 70, the drive plate 72, the drive projection 61 of the drive lever 60, and the rear anchor of the reverse switch 57, certain mechanical strength is required so that these are preferably made of metal such as stainless steel. Moreover, for weight saving of the operating portion 100b, the housing 50 is preferably formed by a synthetic resin such as polycarbonate.

The liquid supplied by the stent delivery catheter 100 of the embodiment includes, but not specifically limited to, a low-viscosity liquid capable of being extruded by the plunger 80, it can be suitably changed depending on the position and purpose for indwelling the stent 40. More particularly, not only normal saline only aiming for enhancing lubricity of the outer tube 20 and biocompatible lubricating oil like silicone oil or olive oil, but also a liquid having other purposes in combination with lubricity: such as a medical agent to be administered to the stent indwelling position or the solution thereof, may be adequately used. In the actual clinical practice, medical agent is often required to be administered to the stent indwelling position. In order to do so, a technology where the medical agent is placed around the stent to administer the agent at the stent indwelling position at a time of stent release is well known. Nevertheless, the technology has a probability such as decidual of the medical agent from the stent when inserting the catheter and falling of the medical agent from the stent when the outer tube is moved towards the proximal end. When the stent delivery catheter 100 of the embodiment is used, after inserting the catheter 100, medical agent can be administered from the distal end 21 of the outer tube 20 to inside the body; thereby it is possible to safely and accurately administer the medical agent to the targeted position compared with the conventional one.

Examples of the medical agent to be used include immunosuppressive agent such as rapamycin, anticoagulant such as heparin, anticancer agent such as 5-FU (5-fluoro uracil), and gene-based medicine.

The stent 40 to be fitted to the stent delivery catheter 100 of the embodiment is not particularly limited to, normally, a self-expandable stent is used. The self-expandable stent means the one expanding from the shrunk state by the self-elastic force; it is usually constituted by a superelastic metal or a shape-memory metal such as nickel-titanium alloy or cobalt-chromium alloy.

When the stent delivery catheter 100 of the embodiment is used for indwelling a self-expandable stent, the liquid supplied from the operating portion 100b into the gap between the inner tube 10 and the outer tube 20 is discharged from the distal end 21 of the outer tube 20 to outside of the catheter 100. During the repeating pivotal operation of the drive lever 60 until the outer tube 20 becomes completely apart from the stent 40, distal end 21 of the outer tube 20 is always in contact with the stent 40, so, each time when the drive lever 60 is operated, the stent 40 is exposed all the time by the liquid which is freshly supplied from the cylinder 58 of the operating portion 100b located outside the body. A self-expandable stent, in general, has a feature in which the expansive force is weaken with a decrease of temperature. Being exposed by the liquid, injected from outside of the body, of which temperature is relatively lower than that of inside the body, a force pressing the inner circumferential surface of the outer tube 20 becomes weaken. Accordingly, it is capable of obtaining effect which lowers friction between the inner tube 10 and the outer tube 20 as well as the catch, but also capable of obtaining secondary effect which lowers friction between the stent 40 and the outer tube 20 with the catch.

As shown in Figs. 2 and 3, at the proximal end of operating portion 100b, a reverse switch 57 is fitted. At the rear anchor portion of the reverse switch 57, a through-hole, through which the drive shaft 70 penetrates in the axial direction, is formed and an end of the pressure spring 67 is abutted thereto. In a state where the reverse switch 57 is not operated, the through-hole of the rear anchor portion of the reverse switch 57 tilts to the axial core of the drive shaft 70, so inner edge of the through-hole latches to the outer circumferential portion of the drive shaft 70 so as to control the movement of the drive shaft 70 in the axial direction. Meanwhile, if the reverse switch 57 is pressed in the arrow B direction, the through-hole becomes substantially perpendicular to the axial core of the drive shaft 70; hence, latch between the inner edge of the through-hole and the drive shaft 70 is released to make the drive shaft 70 be movable to the through-hole of the rear anchor portion of the reverse switch 57 in the axial direction. After use of the catheter 100 and other occasion, if the position of outer tube 20 is required to move back to the distal end direction, it can be carried out by pushing the manual grip 73 back to the direction of catheter portion 100a while pressing the reverse switch 57.

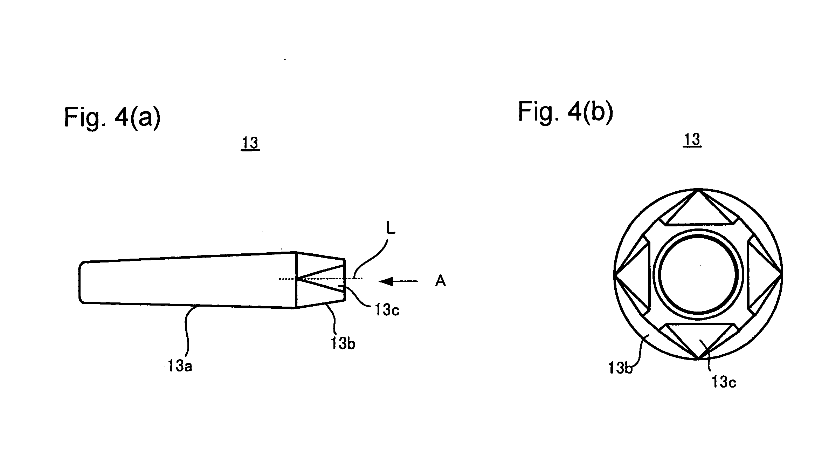

Fig. 4(a) is a side view showing the projection 13 of the stent delivery catheter 100 of Fig. 1; Fig. 4 (b) is a plan view of the projection 13 seen from a direction indicated by arrow A of Fig. 4 (a). The projection 13 of the embodiment, as described above, comprises: a taper portion 13b of which radius is outwardly widen relative to the distal end direction; and an apical portion 13a located at the distal end side of the taper portion 13b and formed in a tapered shape of which radius becomes thinner relative to the distal end direction. In the surface of the taper portion 13b, as shown in Figs. 4 (a) and 4(b), the boundary of the apical portion 13a and the taper portion 13b is determined as a vertex; four of the grooves 13c, which extend in the proximal end direction along the axial direction of the inner tube, are formed at equal space along the circumferential direction. By these grooves, surface of the taper portion 13b is notched in shape of isoscles triangle in a planar view. It should be noted that shape of these grooves 13c in the embodiment are substantially the same.

The tapered shape of the apical portion 13a of the projection 13 decreases mainly insert resistance and that allows the catheter 100 to be easily inserted into the body; meanwhile, tapered shape of the taper portion 13b prevents catch of the projection 13 to the stent 40 when the catheter 100 is removed from the body after the completion of stent indwelling operation.

By providing the taper portion 13b to the projection 13 and removing the step at the distal end of the thin diameter part 11a, it is possible to prevent the catch to the stent 40. However, as a result, when the taper portion 13b is covered by the edge of a distal end opening of the outer tube 20, surface of the taper portion 13b is tightly covered by the outer tube 20; thereby, contact area of the projection 13 and the outer tube 20 increases. The projection 13 and the outer tube 20 are usually formed by a relatively elastic and adhesive material such as resin and elastomer; so, the contact area increases, the inner tube 10 and the outer tube 20 are strongly adhered each other. Consequently, during the moving operation of the outer tube 20 towards the proximal end side, a strong force becomes necessary for removing the outer tube 20 from the projection 13, which cause a strong retroaction at a time when the adhesion is released. And, due to the retroaction, indwelling position of the stent 40 tends to be misaligned. Therefore, as seen from this embodiment, by providing the taper portion 13b, catch to the stent 40 can be prevented, moreover, by providing grooves 13c in the taper portion 13b for reducing the surface area of the taper portion 13b, it is possible to reduce the contact area of the taper portion 13b and the outer tube 20 but also to decrease the adhesion; when the outer tube 20 is moved to the proximal end side as a stent indwelling operation, the outer tube 20 can be smoothly removed from the taper portion 13b.

Shapes and number of the grooves 13c to be provided in the taper surface of the taper portion 13b is not limited to, in view of preventing catch at the distal end opening of the outer tube 20 to the taper portion 13b, the grooves 13c are discontinuously provided in the circumferential direction of the taper portion 13b, each groove 13c is independently formed in a shape extended along the axial direction. The phrase "a shape extended along the axial direction" means a shape where the center line in the longitudinal direction (dotted line L in Fig. 4(a)) in the side view of a groove 13c is parallel to the axial core of the catheter 100. On the other hand, in the plan view (see Fig. 4(b)), it is the shape where the grooves 13c are arranged radially from the axial core of the catheter 100 to the circumferential direction. In order to carry out a smooth moving operation of the outer tube 20 to the proximal end side by further reduction of resistance, the shape of grooves 13c in the surface of taper portion 13b is preferably the one where the width becomes expanded from the distal end towards the proximal end side and is preferably continuously extended from the distal end to the proximal end of taper portion 13b. In addition, so as to lower uneven distribution of the adhesion between the taper portion 13b and the outer tube 20, the grooves 13c are preferably plural and arranged at regular intervals in the circumference direction relative to the axial core of the inner tube body as the center. These grooves 13c are preferably formed in the same shape.

As described above, the grooves 13c mainly work to reduce the surface area of taper portion 13b and to reduce the contact area of the taper portion 13b and the outer tube 20. So, the stereoscopic shape of the groove 13c is not limited to as long as each groove 13c has the above-described shape in the surface of taper portion 13b; it may be notched in any kind of shapes such as curved surface, polygonal pyramid shape, and prismatic column shape.

As for a total area of the grooves 13c in the surface of the taper portion 13b, in view of preventing the edge of distal end opening of the outer tube 20 from coming off from the taper portion 13b by the resistance caused at a time of insert into the body and effectively reduce the friction resistance, the total area of the grooves 13c is preferably 20 to 80%, to the surface area of the taper portion 13b in the state having no groove 13c as 100%.

As material of the projection 13, so as to prevent perforation in inner-wall of the body by the inner tube 10, it is preferably a material having rubber elasticity, and a thermoplastic elastomer is particularly preferable. Among them, a thermoplastic elastomer of which shore hardness is 25D to 72D is preferably used. About the shown embodiment, the projection 13 is formed by a polyamide elastomer. In the embodiment, it should be noted that the apical portion 13a and the taper portion 13b are mounted at the distal end of the inner tube 10 as a projection 13 independently formed from the inner tube 10; the apical portion 13a and the taper portion 13b may be integrally formed at the distal end of the inner tube 10 as a part of the inner tube 10. Moreover, between the apical portion 13a and the taper portion 13b, a center portion, which continues to the both and has an area of which thickness is even, may be formed. Length of the apical portion 13a is preferably 1 to 12 mm, and length of the taper portion 13b is preferably 1 to 5 mm. Further, the maximum outer diameter of the projection 13 (namely, in the embodiment, the outer diameter in the boundary between the apical portion 13a and the taper portion 13b) is preferably 1 to 4 mm.

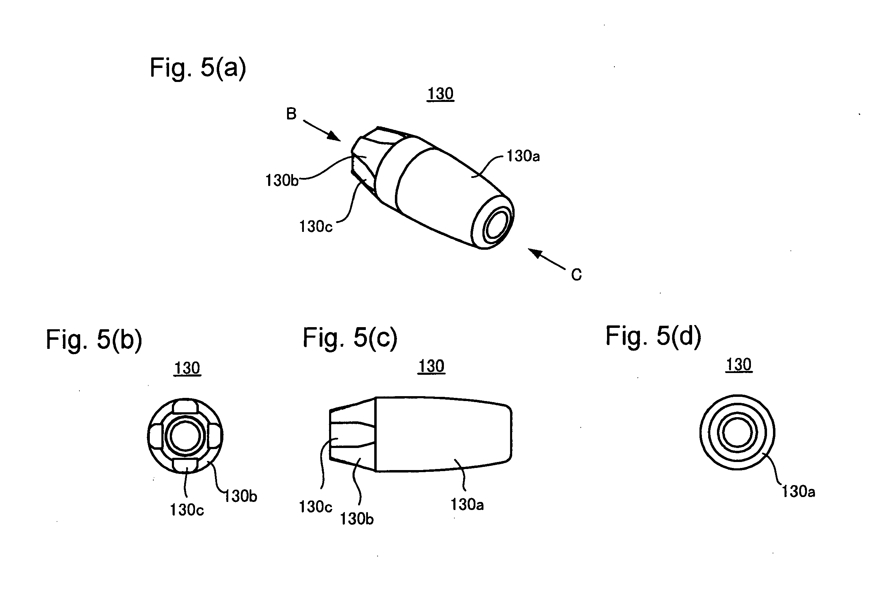

Fig. 5 (a) is a perspective view showing a projection 130 of another embodiment of the stent delivery catheter 100 in Fig. 1; Fig. 5(b) is a plan view of the projection 130 seen from a direction indicated by arrow B; Fig. 5(c) is a side view of the projection 130 of Fig. 5(a); and Fig. 5(d) is a plan view of the projection 130 seen from a direction indicated by arrow C.

The projection 130 comprises: an apical portion 130a formed in tapered shape of which diameter is thinner towards the distal end; and a taper portion 130b which continues to the apical portion 130a and of which diameter is expanded relative to the distal end direction. In the surface of the taper portion 130b of the embodiment, as shown in Figs. 5 (a) and 5(b), four grooves 130c, which are extended in the proximal end direction along the axial direction of the inner tube from a vertex determined at the boundary of the apical portion 130a and the taper portion 130b, are arranged at regular intervals in the circumferential direction. Surface of the grooves 130c is formed in a curved shape. The boundary portion of the apical portion 130a and the taper portion 130b, even at the portion of grooves 130c, is formed without steps.

Number of the grooves is not restricted to, and interval of the grooves is not restricted to the regular interval, either. Ratio of area of the grooves is preferably 20 to 80% to the surface area of the taper portion 130b in the state having no groove 130c as 100%. Except for the shape of the grooves 130c of the projection 130 as seen from Figs. 5 (a) to 5(d), the function as well as shape, property, material, and so on of each constitutional part of the projection 130 are the same as the function as well as shape, property, material, and so on of each constitutional part of projection 13; thereby the description is omitted.

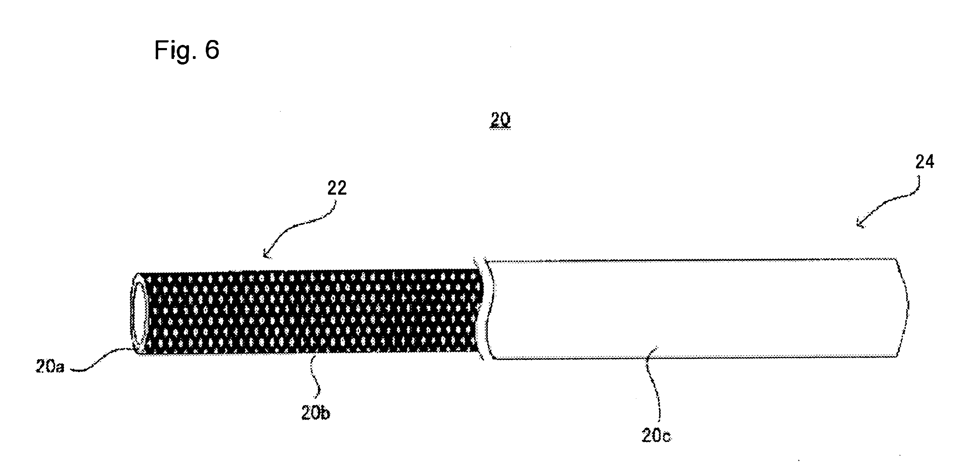

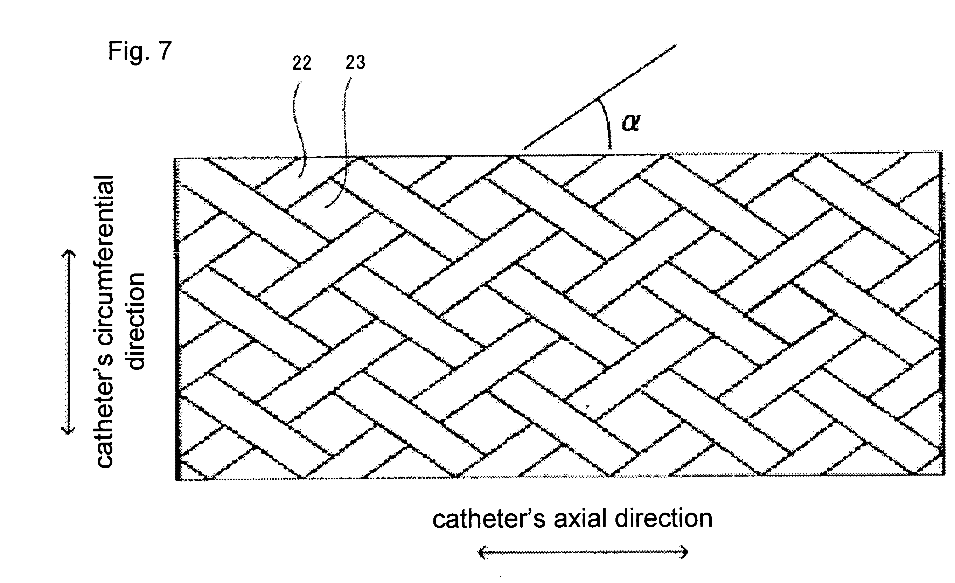

Fig. 6 is a perspective view showing an outer tube 20 of the stent delivery catheter 100 of Fig. 1. In order to clarify the layer composition of the outer tube 20, it is shown by a schematic view in which outer layer of one end is removed. Fig. 7 is an enlarged plan view of a part of tubular braided body 20b included in the outer tube 20.

The outer tube 20 of the embodiment comprises: a resin layer made of a polymer material; and a tubular braided body which is formed by helically-braided a plurality of wire rods and buried in the resin layer, wherein, when the braided body is planarily seen, an angle $\alpha$ (°) between the wire rod and the axis line of inner tube can be determined by the following relational expression.

$$35 < \alpha < 60$$

By having the above feature, it is possible to impart the stiffness for inhibiting kink to the outer tube 20 of the catheter 100; and by observing the image in the monitor through endoscope inserted into the body in parallel with the catheter 100, the operator can accurately recognize the position of the stent 40 inside the outer tube 20.

In should be noted that in Fig. 1 the position for taking a cross-sectional view is set at considerably proximal end side from the tip (distal end) of the outer tube 20. With regard to the longitudinal direction of the outer tube 20, the braided body 20b is not necessarily formed across the entire length of the outer tube 20. Because, existence of the braided body is a disincentive in relation to the transparency of the outer tube 20; meanwhile, in the catheter's apical portion, stiffness such as kink-resistance is not required as much as proximal end side does. Therefore, it is preferable not to arrange the braided body 20b within the length of 1 to 20 cm from the distal end of the outer tube 20. As it were, the braided body 20b is preferably arranged only in the position other than the position equivalent to the stent setting position (thin diameter part 11a in the shown embodiment) of the inner tube 10.

Material constituting the resin layer 24 may be, but not restricted to, a transparent polymer material where the stent 40 is set inside the outer tube 20 can be observed through the outer tube 20. The examples include various resin materials like: polyolefin such as polyethylene and polypropylene; polyester such as polyvinyl chloride, polyurethane, ethylene-vinyl acetate copolymer, polyethylene terephthalate, and polybutylene terephthalate; polyamide; polyether polyamide; polyester polyamide; fluorinated resin such as polytetrafluoro ethylene, or various thermoplastic elastomers like: styrene series, polyolefin series, polyurethane series, polyester series, polyamide series, and polybutadiene series. A combination of two or more thereof may be used.

The resin layer 24 may be formed by laminating two or more layers made of the above polymer materials. The boundary between each layer may be arranged at the position where the braided body 20b exists; it may also be arranged in other areas. In the embodiment shown in Fig. 6, the resin layer 24 is configured with layers being laminated such that the respective layers made of different materials each other sandwich the braided body 20b as a boundary. The resin layer 24 comprises an inner layer 20a placed at the inside the braided body 20b and an outer layer 20c placed outside the braided body 20b.

To the polymer materials which form the resin layer 24, if necessary, various additives such as alloying agent, compatibilizing agent, curing agent, stabilizer, and coloring agent may be mixed, in order to fluoroscopically recognize the tube of catheter inside the body, radiopaque material may be mixed to a part of the tube in the longitudinal direction. Examples of the radiopaque material include tungsten, bismuth oxide, gold, and platinum.

The braided body 20b is configured by braiding a plurality of metallic wire rods 22 and is formed in a tubular body as a whole. In the braided body 20b, individual wire rod 22 is formed in helically-wound shape. Also, the braided body 20b is at least buried in the resin layer 24, it can be arranged to be biased at the vicinity of the outer circumferential surface or inner circumferential surface of the resin layer 24. In the embodiment shown in Fig. 7, the braided body 20b is buried at the boundary between the inner layer 20a and the outer layer 20c both of which constitute the resin layer 24.

Material of the wire rod 22 constituting the braided body 20b is not particularly restricted to as long as it is metal. The examples include: metal element such as gold, silver, platinum, copper, iridium, nickel, titanium, tungsten, iron, aluminum, tin, and zinc; and alloy such as stainless steel, nichrome steel, nickel-titanium alloy, and titanium series alloy. Among them, in view of workability, strength, and corrosion resistance, stainless steels stipulated in Japanese Industrial Standard (JIS) SUS 304, SUS 316 and so on may be preferably used. Moreover, a plurality of these metals may be used in combination to constitute the wire rod 22. A plurality of the wire rods 22 may also be individually formed using different metal materials. Further, to the wire rods 2, plating or coating may be given.

In the outer tube 20, tilt angle α between the wire rod 22 constituting the braided body 20b and the longitudinal axis of the outer tube 20 is preferably 35° to 60°, more preferably 40° to 55°, and still further preferably 45° to 50°. If the tilt angle α of the wire rod 22 is too large or too small, the wire rod 22 in the planar view becomes dense so that transparency of the outer tube 20 becomes lower; therefore, accurately recognizing the position of stent 40 inside the outer tube 20 becomes difficult for the operator. In addition, if the tilt angle α is too large, stiffness of the outer tube 20 is lowered so that the tube tends to kink.

About the outer tube 20, number of the wire rod 22 constituting the braided body 20b is not specifically limited to as long as it is plural; it should be determined to satisfy the above individual conditions, even number is preferable. When number of the wire rod 22 is even number, by composing the braided body 20b with the same number of left-handed-helix wire rod 22 and right-handed-helix wire rod 22, it is possible to compose a well-balanced braided body 20b. More specifically, number of the wire rod 22 constituting the braided body 20b is preferably even number in the range within 2 to 32, most preferably 16. Using the wire rods 22 consisting of the above number makes the forming of braided body 20b easier.

When the outer tube 20 is planarily seen as shown in Fig. 7, a mesh 23 of the braided body 20b is preferably rhomboid. By making the mesh 23 of the braided body 20b in such a shape, braided body 20b can obtain a well balance; thereby the outer tube 20 becomes less kinky. In order to make the mesh 23 of braided body 20b in rhomboid shape, number of the left-handed-helix wire rod 22 and the right-handed-helix wire rod 22, both of which compose the braided body, should be determined to be the same and individually placed at regular intervals.

Outer diameter of the braided body 20b is not specifically restricted to; it is selected depending on the usage of the stent delivery catheter 100 constituted by the outer tube 20, normally, it is 1.6 to 3.6 mm, preferably 1.9 to 3.0 mm. Thickness of the outer tube 20 is not also restricted to; when the thickness of the outer tube 20 is relatively thin, the stent delivery catheter 100 of the present invention exhibits notably excellent effect compared with the conventional catheter tube. So, the thickness thereof may preferably be 0.03 to 0.35 mm, particularly preferably 0.05 to 0.20 mm.

The outer tube 20 can be manufactured in accordance with the manufacturing method of a catheter tube (the so-called "blade tube") in which a known braided body is buried. More specifically, a method, in an order specified, comprising the steps of: forming an inner layer 20a to be arranged inside a braided body 20b in advance; forming a braided body 20b by braiding with winding wire rods 22 around the outer circumferential surface of this tube 20a by a braiding machine; and then, extruding an outer layer 20c to be arranged outside the braided body 20b, to the outer circumferential surface. The outer tube 20 can also be manufactured in accordance with the method comprising the steps of: forming a braided body 20b in advance; and forming a resin layer 24 by impregnating the braided body 20b in a resin solution.

The position in the body for indwelling the stent 40 by the stent delivery catheter 100 described above is not specifically limited to. It can be used in various celoms like digestive organ such as esophagus, duodenum, bile duct, small intestine, large intestine; urinary system such as ureter, urethra; trachea, blood vessel, or the like, for indwelling the stent 40. Specifically, the stent delivery catheter 100 of the present invention can be suitably used for indwelling the stent 40 to digestive organs through endoscope. Still further specifically, when it is used for indwelling the stent 40 to a bile duct which has many bending and branches which makes indwelling operation of the stent 40 difficult, it is possible to make the most of the effect (in easiness of operation) of the catheter 100 of the invention; thereby the stent delivery catheter 100 of the present invention is particularly effective.

Embodiments

The outer diameter of outer tube, pitch of the wire rod constituting the braided body, and tilt angle α are varied to evaluate strength and visibility of the outer tube (transparency). The results are shown in Table 1.

(Table 1)

|  | Diameter of tube (φ) | Pitch (mm) | Angle α (°) | Strength of tube (N) | Visibility of outer tube |
|---|---|---|---|---|---|
| Outer tube A | 2.7 | 7 | 50 | 72.9 | high |
| Outer tube B | 2.7 | 1 | 83 | 35.6 | low |
| Outer tube C | 2.7 | 3 | 70 | 55.5 | low |

As for the outer tube A as the preferable embodiment of the invention of which tilt angle α between the wire rod and the axis line of inner tube is larger than 35° and smaller than 60°, stiffness (strength) was maintained and transparency (visibility) was also appropriate. On the contrary, the outer tube B and C of which tilt angle α is larger than the preferable range of the present invention showed inferior results in stiffness (strength) and transparency (visibility) to the Example of the present invention.

**Claims**

1.  A stent delivery catheter comprising:

    an inner tube which has a distal end and a proximal end and in which a stent is set around the outer circumferential surface at the distal end side of said inner tube;
    an outer tube having a distal end and a proximal end, being coaxially arranged around the outer circumference of said inner tube, and being relatively movable in the axial direction to said inner tube; and
    a drive means being arranged at the proximal end side of said inner tube and said outer tube and being capable of making said outer tube relatively movable towards the proximal end to said inner tube,
    wherein with the operation of said drive means, a liquid fluidly travels through the gap between said inner tube and said outer tube from the proximal end side to the distal end direction.

2.  The stent delivery catheter according to claim 1 further comprising a housing which incorporates the proximal end of said inner tube and the proximal end of said outer tube, in which said outer tube is relatively movably inserted in the axial direction, and to which said drive means is mounted,
    wherein, in said housing, a cylinder for reserving the liquid is arranged, and a plunger is relatively movably arranged to said cylinder in the axial direction of said cylinder and fixed to said outer tube,
    when said outer tube moves relatively to said inner tube in the proximal end direction by the motion of said drive means, said plunger presses the liquid in said cylinder; thereby the liquid travels, by the pressure, through the gap between said inner tube and said outer tube from the proximal end side to the distal end direction.

3.  The stent delivery catheter according to claim 1 further comprising a housing which incorporates the proximal end of said inner tube and the proximal end of said outer tube, in which said outer tube is relatively movably inserted in the axial direction, and to which said drive means is mounted,
    wherein said drive means comprises: a drive shaft fixed at a proximal end portion of said outer tube and being relatively movable in the axial direction to said housing;
    a drive plate having a through-hole, being movably held in the axial direction to said drive shaft by inserting said drive shaft into said through-hole, and being latched to said drive shaft by tilting towards the axial core of said drive shaft; and
    a drive lever which is pivotaly mounted around a fulcrum located at a position certain distance away from the axial core of said housing and which is pressed, by pivoting, towards the proximal end side of said drive plate,
    wherein said drive shaft makes said drive plate be tiltedly latched onto said drive lever by pressing said drive plate towards the proximal end side, and further makes both said drive shaft and said outer tube fixed to said drive shaft be moved towards the proximal end side together with said drive plate.

4. The stent delivery catheter according to claim 1, wherein said inner tube comprises a cylindrical inner tube body and a projection arranged at the distal end side of said inner tube body and projecting from the distal end of said outer tube,
said inner tube body comprises a cylindrical body portion and a thin diameter part arranged at the distal end side of said body portion and formed in a manner thinner than the diameter of said body portion for arranging said stent, said projection comprises: a taper portion which is continuously arranged with the thin diameter part and has a taper expanding the radius relative to the distal end direction; and an apical portion located at the distal end side of the taper portion,
on the surface of said taper portion, groove extending along the axial direction of said inner tube is formed.

5. The stent delivery catheter according to claim 4, wherein said groove is formed such that the width is widened towards the proximal end direction.

6. The stent delivery catheter according to claim 4, wherein said groove is continuously extended from the distal end to the proximal end of said taper portion.

7. The stent delivery catheter according to claim 4, wherein said groove is plurally provided.

8. The stent delivery catheter according to claim 7, wherein a plurality of said grooves are arranged at regular intervals in the circumference direction relative to the axial core of the inner tube body as the center.

9. The stent delivery catheter according to claim 7, wherein a plurality of said grooves are in the same shape.

10. The stent delivery catheter according to claim 4, wherein said inner tube body and said projection are independent from each other.

11. The stent delivery catheter according to claim 1, wherein said outer tube comprises: a resin layer made of a polymer material; and a tubular braided body which is formed by helically-braided plurality of wire rods and buried in the resin layer,
wherein, when said braided body is planarily seen, an angle $\alpha$ (°) between said wire rod and said axis line of inner tube can be determined by the following relational expression.

$$35 < \alpha < 60$$

12. The stent delivery catheter according to claim 11, wherein said braided body is arranged in the position other than the position corresponding to the stent setting position in said inner tube.

# Fig. 1(a)

<u>100</u>

# Fig. 1(b)

<u>100a</u>

EP 2 002 808 A2

Fig. 2

100b

EP 2 002 808 A2

Fig. 3

100b

EP 2 002 808 A2

Fig. 4(a)

Fig. 4(b)

Fig. 5(a)

B

130

130b

130c

130a

C

Fig. 5(b)

130

130c

130b

Fig. 5(c)

130

130c 130b

130a

Fig. 5(d)

130

130a

EP 2 002 808 A2

Fig. 6

Fig. 7

22   23

$\alpha$

catheter's circumferential direction

catheter's axial direction

EP 2 002 808 A2

**EP 2 002 808 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004223262 A
- US 20040148007 A
- JP 10057502 A
- EP 0819411 A
- JP 11313893 A
- US 6425898 B
- JP 2001037885 A
- EP 1064888 A